# EUROPEAN PATENT APPLICATION

(11) **EP 4 520 320 A2**
(43) Date of publication of application: **12.03.2025**
(21) Application number: 24212922.9
(22) Date of filing: 22.02.2017
(51) Int. Cl.: A61K 9/00

(54) **IMPROVED BLADDER INJECTION PARADIGM FOR ADMINISTRATION OF BOTULINUM TOXINS**

(30) Priority: 22.02.2016 US 201662298309 P
(62) Divisional of application: 22159131.6
(71) Applicant: Allergan, Inc., Irvine, CA 92612 (US)
(72) Inventor: Alvandi, Nancy, Sausalito, California, 94965 (US); Dadas, Christopher A., Strongsville, Ohio, 44136 (US); Joshi, Manher A., Santa Ana, California, 92707 (US)
(74) Representative: Hoffmann Eitle

(57) **Abstract**

Methods for treating a bladder dysfunction by injecting a clostridial derivative to a target site below the bladder midline are described.

## Description

### Cross-Reference to Related Applications

This application claims the benefit of U.S. Provisional Application No. 62/298,309 filed February 22, 2016, incorporated herein by reference in its entirety.

### Technical Field

The present disclosure relates to a method for treating overactive bladder dysfunction by local administration of a clostridial neurotoxin. In particular, the present disclosure relates to an improved injection paradigm for administration of a clostridial neurotoxin to the bladder.

### Background

Neurotoxin therapies, in particular botulinum toxins, have been used for the treatment of various medical conditions, including urological conditions such as overactive bladder (OAB) and detrusor overactivity. The normal bladder has two functions, storage and voiding of urine. Problems affecting urine storage are common and result in urinary urgency, increased frequency of urination, nocturia and urgency incontinence. These symptoms are commonly associated with spontaneous involuntary contraction of the detrusor muscle and are associated most commonly with a condition referred to as overactive bladder. Overactive bladder can be caused by a variety of neurological and non-neurologic conditions, or develop from an unknown cause.

Botulinum toxin therapy to treat bladder disorders such as overactive bladder, detrusor overactivity associated with a neurological condition, is typically administered by injection across the urinary bladder wall and into the innervated muscular tissues surrounding the bladder. Botulinum toxin has been shown to reduce the numbers of incontinence episodes, reduce urinary urgency, reduce urinary frequency and nocturia, increase bladder capacity and reduce involuntary bladder contractions.

Some of the common side effects associated with botulinum toxin injection into the bladder include urinary tract infections, dysuria (painful voiding), and urinary retention. Urinary retention is defined as the inability to completely or partially empty the bladder. Symptoms of urinary retention include difficulty starting urination, difficulty completely emptying the bladder, weak urine stream, decreased sensation of bladder fullness, need to strain to empty bladder (valsava), and the feeling of frequently needing to empty the bladder. If left untreated, urinary retention can lead to potentially serious and life-threatening complications such as urinary tract infections, pyelonephritis, and ultimately kidney damage. A well accepted treatment for urinary retention is clean intermittent catheterization (CIC), where a patient places a small tube into their urethra in order to drain the bladder. The risk of urinary retention and the need to perform CIC can have a negative impact on the quality of life of patients and may cause the patient to not undergo future treatments.

Thus, there is a need for an improved administration paradigm of botulinum toxin for treatment of urological conditions that reduces the risk for urinary retention, and consequently has an improved safety profile and enhanced efficacy.

### Summary

Aspects of the present disclosure provides a method for treating a bladder dysfunction or detrusor overactivity in a patient in need thereof, the method comprising locally administering a composition comprising a therapeutically effective amount of a clostridial derivative to a target site below (or inferior to) the bladder midline. In some embodiments, the target site comprises the bladder base, the posterior and lateral bladder wall, or both. In some embodiments, the target site below (or inferior to) the bladder midline is selected from the regions consisting of the bladder base, the posterior and lateral bladder wall, the bladder base exclusive of the trigone, the bladder base exclusive of the trigone and the bladder neck, the trigone only, and the bladder neck only.

In one embodiment, the bladder midline corresponds to an imaginary approximately 2-3 cm above an imaginary line intersecting the trigone above the ureteral orifices.

In some embodiments, the bladder dysfunction is an overactive bladder. In alternative embodiments, the detrusor overactivity is associated with a neurologic condition. In some embodiments, the clostridial derivative is a botulinum toxin. In some embodiments, the clostridial derivative is a botulinum toxin type A.

In another aspect, the present disclosure provides a method for reducing or preventing the risk for urinary retention associated with overactive bladder or neurogenic detrusor overactivity treatment using a clostridial derivative in a patient. The method comprises locally administering a composition comprising a therapeutically effective amount of the clostridial derivative to a target site below (or inferior to) the bladder midline.

In another aspect, the present disclosure provides a method for reducing the need for clean intermittent catherization (CIC) associated with overactive bladder or neurogenic detrusor overactivity treatment using a clostridial derivative in a patient. The method comprises locally administering a composition comprising a therapeutically effective amount of the clostridial derivative to a target site below (or inferior to) the bladder midline.

In another aspect, by alleviating one or more adverse events or side effects associated with clostridial treatment of overactive bladder or detrusor overactivity in a patient in need thereof, the present methods provide functional improvement and thus improve the quality of life for the patient.

### Brief Description of the Figures

The following drawings are presented to illustrate aspects and features of embodiments of the present invention.
FIG. 1 is a cross-sectional view of a bladder and shows certain anatomical landmarks and a prior art bladder injection pattern for treatment of overactive bladder and of detrusor overactivity;
FIG. 2A is a cross-sectional view of a bladder and shows a bladder injection pattern in accordance with aspects of the present disclosure;
FIGS. 2B-2C show administration via injection to the detrusor muscle; and
FIGS. 3A-3B are diagrams of a bladder in a cross-sectional view comparing the prior art injection pattern (FIG. 3A) and the injection pattern in accordance of the present disclosure (FIG. 3B).

### Description

### Definitions

### The following definitions apply herein:

"About" or "approximately" as used herein means within an acceptable error range for the particular value as determined by one of ordinary skill in the art, which will depend in part on how the value is measured or determined, (*i.e.,* the limitations of the measurement system). For example, "about" can mean within 1 or more than 1 standard deviations, per practice in the art. Where particular values are described in the application and claims, unless otherwise stated, the term "about" means within an acceptable error range for the particular value.

"Administration", or "to administer" means the step of giving (i.e. administering) a botulinum toxin to a subject, or alternatively a subject receiving a pharmaceutical composition.

"Alleviating" means a reduction in the occurrence of a pain or other symptoms associated with bladder overactivity. Thus, alleviating includes some reduction, significant reduction, near total reduction, and total reduction. An alleviating effect may not appear clinically for between 1 to 7 days after administration of a clostridial derivative to a patient or sometimes thereafter.

"Botulinum toxin" means a neurotoxin produced by Clostridium botulinum, as well as a botulinum toxin (or the light chain or the heavy chain thereof) made recombinantly by a non-Clostridial species. The term "botulinum toxin", as used herein, encompasses the botulinum toxin serotypes A, B, C, D, E, F and G, and their subtypes and any other types of subtypes thereof, or any re-engineered proteins, analogs, derivatives, homologs, parts, sub-parts, variants, or versions, in each case, of any of the foregoing. "Botulinum toxin", as used herein, also encompasses a "modified botulinum toxin". Further "botulinum toxin" as used herein also encompasses a botulinum toxin complex, (for example, the 300, 600 and 900kDa complexes), as well as the neurotoxic component of the botulinum toxin (150 kDa) that is unassociated with the complex proteins.

"Clostridial derivative" refers to a molecule which contains any part of a clostridial toxin. As used herein, the term "clostridial derivative" encompasses native or recombinant neurotoxins, recombinant modified toxins, fragments thereof, a Targeted vesicular Exocytosis Modulator (TEM), or combinations thereof.

"Clostridial toxin" refers to any toxin produced by a Clostridial toxin strain that can execute the overall cellular mechanism whereby a Clostridial toxin intoxicates a cell and encompasses the binding of a Clostridial toxin to a low or high affinity Clostridial toxin receptor, the internalization of the toxin/receptor complex, the translocation of the Clostridial toxin light chain into the cytoplasm and the enzymatic modification of a Clostridial toxin substrate.

"Effective amount" as applied to the biologically active ingredient means that amount of the ingredient which is generally sufficient to induce a desired change in the subject. For example, where the desired effect is a reduction in calculi formation, an effective amount of the ingredient is that amount which causes at least a substantial reduction of bladder overactivity and associated symptoms, and without resulting in significant toxicity.

"Implant" means a controlled release (e.g., pulsatile or continuous) composition or drug delivery system. The implant can be, for example, injected, inserted or implanted into a human body.

"Local administration" means administration of a pharmaceutical agent at or to the vicinity of a site on or within an animal body, at which site a biological effect of the pharmaceutical is desired, such as via, for example, intramuscular or intra- or subdermal injection or topical administration. Local administration excludes systemic routes of administration, such as intravenous or oral administration. Topical administration is a type of local administration in which a pharmaceutical agent is applied to a patient's skin.

"Peripheral administration" means administration to a location away from a symptomatic location, as opposed to a local administration.

"TEMs", abbreviated for Targeted Exocytosis Modulators are retargeted endopeptidases that direct the catalytic activity of the light chain to specific types of neuronal cells or to target cells that were not affected by botulinum toxins expanding the beneficial clinical effect of inhibition of exocytosis in several human diseases.

"Treating" or "treatment" means to alleviate (or to eliminate) at least one symptom (such as, for example, hip and groin pain), either temporarily or permanently.

"Therapeutically effective amount" refers to an amount sufficient to achieve a desired therapeutic effect.

### Methods of Treatment

In one aspect, a method for treating a bladder dysfunction or detrusor overactivity in a patient in need thereof. The method comprising locally administering a composition comprising a therapeutically effective amount of a clostridial derivative to a target site below (or inferior to) the bladder midline. In some embodiments, the target site comprises the bladder base, the bladder neck only, the trigone only, the posterior and lateral bladder wall, or combinations thereof.

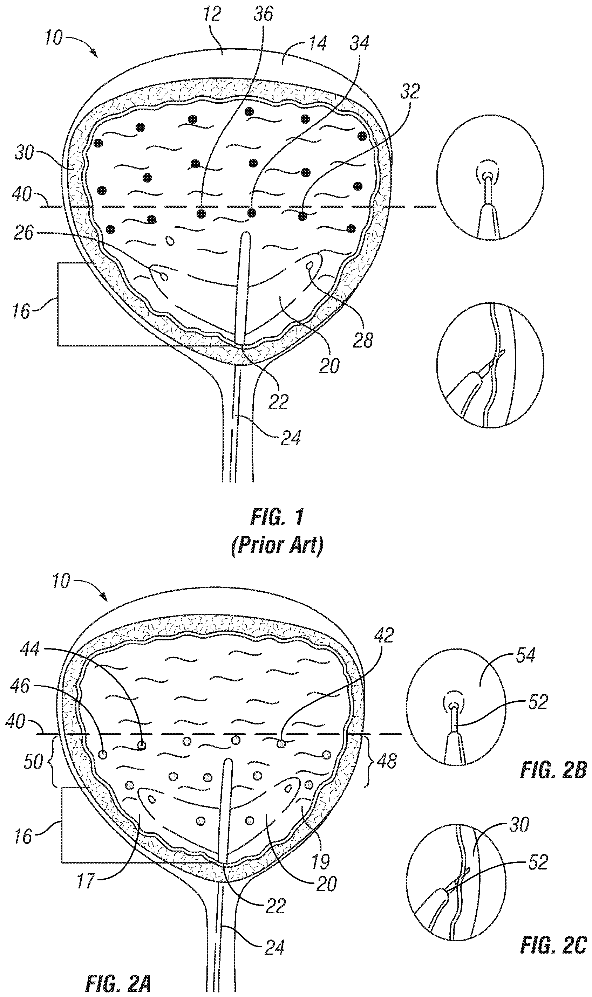

With reference to **FIG. 1****,** a frontal cross-sectional view of a bladder **10** is shown. The hollow organ has a vertex or apex **12,** a superior surface **14** (also referred to as the dome), and an inferior surface or base **16.** Base **16** comprises the posteriorly and inferiorly facing surfaces of the organ. The trigone **20** lies at (and within) the base of the bladder and borders the posterior side of bladder neck **22.** Bladder neck **22** is within the bladder base and corresponds to a region where the walls of the bladder converge and connect with the urethra **24.** At the lateral points, designated as **26, 28,** of trigone **20** the ureters empty into the bladder cavity through the ureteral orifices. The detrusor muscle **30** is a layer in the bladder wall of smooth muscle fibers.

With continuing reference to **FIG. 1****,** a prior art injection pattern for treatment of overactive bladder and of detrusor overactivity is shown by the pattern of injection sites represented by sites **32, 34,** and **36.** In this prior art injection pattern, the injection sites are concentrated in the upper portion of the bladder, well above bladder base **16.** With reference to a midline of the bladder, denoted in **FIG. 1** by dashed line **40,** the injection sites in the prior art injection pattern are substantially at or above bladder midline **40.** In the prior art injection pattern between 20 and 30 injection sites are recommended, the sites spaced approximately 1 cm apart (BOTOX^{®} Package Insert). The injection pattern, shown in FIG. 1, concentrates the injection sites at or above the bladder midline, where more than 70% or 80% of the injection sites are at or above the bladder midline.

In the present methods, treatment is achieved by administration of a clostridial derivative to a target site below or inferior to the bladder midline. With reference to **FIG. 2A****,** the same cross-sectional view of the bladder discussed in **FIG. 1** is shown, now where a target site for administration is at or below bladder midline **40.** In the embodiment shown in **FIG. 2A****,** the target site comprises a plurality of injection sites, represented by sites **42, 44, 46,** that form an injection pattern. In this embodiment, 80% or more of the injection sites in the plurality are at or inferior to bladder midline **40.** In another embodiment, 90% or more of the injection sites in the plurality are at or inferior to bladder midline **40.** In another embodiment, each of the injection sites in the plurality are at or inferior to bladder midline **40.** In another embodiment, 80%, 90% or 100% of the injections sites in the plurality of injection sites are inferior to the bladder midline. By way of example, 20 injection sites form a recommended injection paradigm. In accord with one embodiment of the method, 16 of the 20 injection sites (80% or more) are at or below the bladder midline. In accord with another embodiment of the method, 16 of the 20 injection sites (80% or more) are below the bladder midline.

In another embodiment of the method, the target site for administration to the bladder is bladder base **16,** only the bladder neck **22,** only trigone 20, and/or the posterior and lateral bladder wall below the bladder midline (**40**) designated in **FIG. 2A** by regions **48, 50.** The band of bladder tissue spanning the regions designated as **48, 50** are referred to herein as the postero-lateral bladder wall or the posterior and lateral bladder wall.

In one embodiment, and as seen in **FIG. 2A****,** bladder base **16** is comprised of trigone **20,** bladder neck **22,** and regions of the postero-lateral bladder wall designated at **17** and **19** that are outside the lateral edges of the trigone and below the ureteral orifices. In another embodiment, the target site for administration is the bladder base exclusive of the trigone. In another embodiment, the target site for administration is the bladder base exclusive of the bladder neck. In one embodiment, the target site comprises the bladder base exclusive of the trigone and the bladder neck. In another embodiment, the target site comprises only the trigone. In other embodiments, the target site is only the bladder neck; stated alternatively, the target site is the bladder base exclusive of the trigone and the postero-lateral bladder wall designated at **17** and **19.** In one embodiment, the target site is the bladder base and the postero-lateral bladder wall below the bladder midline.

As discussed elsewhere herein, the injection paradigm is contemplated for use in treating bladder dysfunction, including overactive bladder and detrusor over activity associated with a neurologic condition. Treatment at the target site with a clostridial derivative is administered, typically by injection, to detrusor muscle **30.** This is illustrated in **FIGS. 2B-2C** where a needle **52** configured to deliver the clostridial derivative penetrates bladder wall **54** into detrusor muscle **30.** Accordingly, in embodiments where the target site is the bladder base, the clostridial derivative is administered in one embodiment by injection to the detrusor muscle. In embodiments where the target site is the bladder neck the clostridial derivative is administered, in one embodiment, by injection to the detrusor muscle. In embodiments where the target site is the posterior and lateral bladder wall below the bladder midline the clostridial derivative is administered, in one embodiment, by injection to the detrusor muscle.

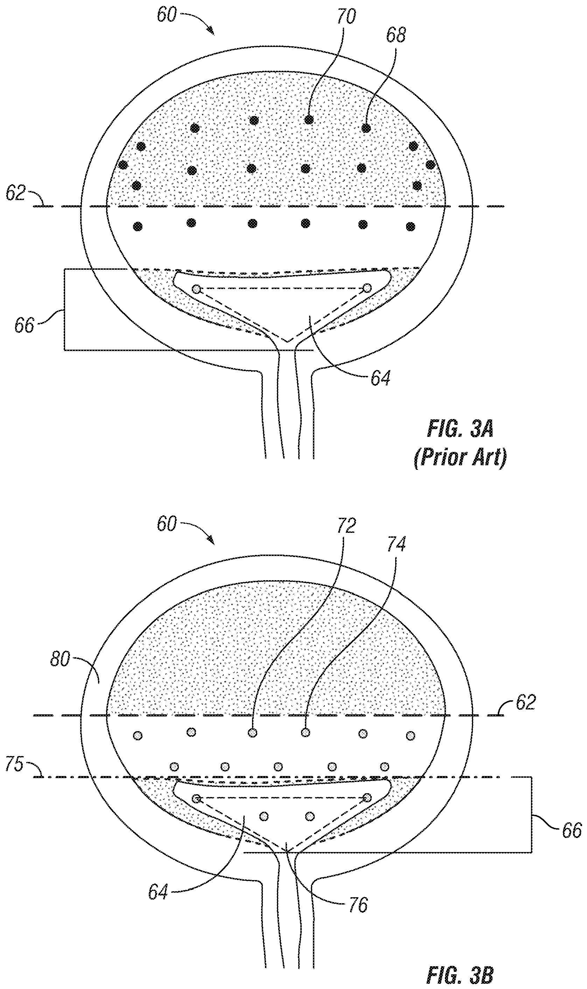

**FIGS. 3A-3B** are diagrams showing a cross-sectional view of a bladder **60** comparing the prior art injection pattern (FIG. 3A) and the injection pattern in accordance of the present disclosure (FIG. 3B). Bladder **60** is intersected by a midline, shown by dashed line **62,** with a bladder portion above the midline and a bladder portion the midline. In the bladder portion below midline **62** is the trigone **64** which is within a region referred to herein as the bladder base, denoted in **FIGS. 3A-3B** by the shaded area identified at **66.** The prior art injection pattern illustrated in **FIG. 3A** comprises a plurality of injection sites, designated by the dots with dots **68, 70** representative, where the plurality is substantially at or above the bladder midline. That is the injection sites in the prior art injection pattern are primarily at or above the bladder midline with more than 65%, 70% or 80% of the injection sites are at or above the bladder midline.

As seen in **FIG. 3B****,** in the injection pattern disclosed herein, the target site for administration is at or below the bladder midline, and preferably below the bladder midline. Injection sites **72, 74,** representative of a plurality of injection sites, are positioned below the bladder midline. In one embodiment, 80% or more, 85% or more, 90% or more, 95% or more, or 100% of the injection sites are below the bladder midline. In one embodiment, each injection site in the plurality is outside of trigone **64.** Stated alternatively, the target site for administration excludes the trigone, or the injection pattern defined by the injection site(s) excludes the trigone. In another embodiment, the target site for administration is the posterior and lateral bladder wall below the bladder midline and/or bladder base **66,** inclusive or exclusive of bladder neck **76.** In one embodiment, the target site for administration is the trigone 64. In one embodiment, the clostridial derivative is administered by injection at the target site (which in all embodiments is below the bladder midline) to detrusor muscle **80.**

In one embodiment, the bladder midline corresponds to an imaginary line or plane approximately 2-3 cm above the trigone. In one embodiment, the bladder midline corresponds to an imaginary line or plane approximately 2-3 cm above an imaginary line **75** that intersects the edges of the trigone above the ureteral orifices (see **FIG. 3B**). In another embodiment, the bladder midline corresponds to an imaginary line or plane that divides the bladder into two sections of substantially equal volume. In another embodiment, the bladder midline corresponds to an imaginary line or plane that divides the bladder into two sections of substantially equal surface area. In another embodiment, the bladder midline corresponds to an imaginary line or plane that divides the bladder into approximately two equal halves of an upper half and a lower half in the judgment of a medical provider.

Without being bound by theory, a physiological mechanism can be set forth to explain the efficacy of the present disclosure. The bladder is comprised of many types of cells. Recently established are the presence of bladder interstitial cells (ICs) which appear to share some of the properties of the pacemaker ICs in the gut (Drake, M.J. et al., J Urol. 2003;169:1573-6; McCloskey, K.D. et al., J Urol. 2002;168:832-6; Hashitani, H. et al., J Physiol. 2004;559:567-81). Like their counterparts in the gut, bladder ICs are thought to play a role in bladder function and dysfunction (Drake, M.J., Ann R Coll Surg Engl. 2007;89(6):580-5). In the gut, ICs help regulate peristalsis by: 1) segmentation contractions or 2) propulsive contractions. In segmentation contractions a back-and-forth squeezing mixes the gut content with the digestive enzymes (Id.). Segmentation contractions arise because the gut wall is arranged in functional modules (Furness, J.B. and Costa, M. The enteric nervous system. Edinburgh: Churchill Livingstone; 1987), which are areas capable of independent, localized contraction, In propulsive contractions, a contraction wave propagates along the gut wall to push the contents to the next part of the bowel. In the bladder, spontaneous localized and propagating contractions can be observed, somewhat similar to gut peristalsis (Coolsaet, B.L. et al., Neurourol Urodyn. 1993;12:463-71). Under certain circumstances, the modules might contract independently (equivalent to segmentation) or neighboring modules may become co-ordinated (propagating peristalsis) (Drake, M.J., Ann R Coll Surg Engl. 2007;89(6):580-5). Under normal circumstances, these modules work together to peristaltically contract and void urine from the bladder.

The present method is based, in part, on the peristaltic ability of the bladder and bladder neuronal innervation. Specific injection locations within the bladder are thought to reduce the risk of urinary retention. For example, the majority of bladder innervation is around the lower portion of the bladder, including the trigone. Rather than indiscriminately injecting the detrusor muscle, resulting in chemo denervation of an excessive portion of the bladder involved in expelling urine, the present method focuses on modulating bladder contractions in the inferior portion of the bladder (FIG. 2A and FIG. 3B).

Among other benefits, the present method substantially reduces the risk for urinary retention and the potential need for CIC by at least two mechanisms. First, sparing contractility in the dome and upper portion of the bladder allows for sufficient bladder contraction to maintain voiding function. Second, normal voiding requires a coordinated wave of contraction in the bladder muscle, with relaxation in the internal sphincter (near the bladder base and neck) and external sphincter.

By administrating the clostridial derivative to the bladder base, the bladder neck only, the trigone only, and/or the posterior and lateral bladder wall, the present method allows sufficient relaxation of the detrusor muscle to reduce bladder pressures as well as maintain the afferent sensory reductions without affecting the contractility of the superior portion of the bladder (above the bladder midline). Thus, the present method maintains the peristaltic contractions of the bladder without causing excessive weakness that may result in urinary retention. Administration of the clostridial derivative to the bladder neck among other things, relaxes this portion of the bladder and facilitates unobstructed flow of urine through the urethra.

In another aspect, the present disclosure provides a method for reducing or preventing the risk for urinary retention associated with overactive bladder or neurogenic detrusor overactivity treatment using a clostridial derivative in a patient. The method comprises locally administering a composition comprising a therapeutically effective amount of the clostridial derivative to a target site below (or inferior to) the bladder midline. In some embodiments, the target site comprises the bladder base, the bladder neck only, the bladder base exclusive of the trigone, the trigone only, the posterior and lateral bladder wall below the bladder midline, or combinations thereof. In some embodiments, the target site comprises the bladder neck only. In some embodiments, the target site comprises the posterior and lateral bladder wall. In some embodiments, the target site is the bladder base. In other embodiments, the target site is the bladder base excluding the trigone. In other embodiments, the target site is the bladder base excluding the trigone and the bladder neck. In other embodiments, the target site is only the bladder neck. In other embodiments, the target site is the posterior and lateral bladder wall below the bladder midline. In some embodiments, the target site comprises only the trigone. In some embodiments, the target site is the trigone. In some embodiments, the target site excludes the trigone. In some embodiments, in addition to the bladder base, the clostridial derivative is also administered above the bladder midline.

In some embodiments, the bladder dysfunction is an overactive bladder. In alternative embodiments, the detrusor overactivity is associated with a neurologic condition. In some embodiments, the clostridial derivative is a botulinum toxin. In some embodiments, the clostridial derivative is a botulinum toxin type A.

In some embodiments, the composition is administered by injections, including intramuscular injections or non-intramuscular injections. In alternative embodiments, the administration is topical.

Botulinum neurotoxins (BoNTs) such as, for example, BoNT/A, BoNT/B, etc., act on the nervous system by blocking the release of neurosecretory substances such as neurotransmitters. The action of BoNT is initiated by its binding to a receptor molecule on the cell surface, and then the toxin-receptor complex undergoes endocytosis. Once inside the cell, BoNT cleaves exocytotic specific proteins responsible for neurotransmitter docking and release from the cell known as the SNARE proteins (soluble N-ethylmaleimide-sensitive factor attachment protein receptor). The resulting transient chemodenervation has been utilized medically to block motor neurotransmission at the neuromuscular junction leading to a variety of therapeutic applications.

In some embodiments, the clostridial derivative includes a native, recombinant clostridial toxin, recombinant modified toxin, fragments thereof, TEMs, or combinations thereof. In some embodiments, the clostridial derivative is a botulinum toxin. In some embodiments, the botulinum toxin can be a botulinum toxin type A, type B, type C₁, type D, type E, type F, or type G, or any combination thereof. The botulinum neurotoxin can be a recombinantly made botulinum neurotoxins, such as botulinum toxins produced by *E. coli.* In alternative embodiments, the clostridial derivative is a TEM.

In some embodiments, the botulinum neurotoxin can be a modified neurotoxin, that is a botulinum neurotoxin which has at least one of its amino acids deleted, modified or replaced, as compared to a native toxin, or the modified botulinum neurotoxin can be a recombinant produced botulinum neurotoxin or a derivative or fragment thereof. In certain embodiments, the modified toxin has an altered cell targeting capability for a neuronal or non-neuronal cell of interest. This altered capability is achieved by replacing the naturally-occurring targeting domain of a botulinum toxin with a targeting domain showing a selective binding activity for a non-botulinum toxin receptor present in a non-botulinum toxin target cell. Such modifications to a targeting domain result in a modified toxin that is able to selectively bind to a non-botulinum toxin receptor (target receptor) present on a non-botulinum toxin target cell (re-targeted). A modified botulinum toxin with a targeting activity for a non-botulinum toxin target cell can bind to a receptor present on the non-botulinum toxin target cell, translocate into the cytoplasm, and exert its proteolytic effect on the SNARE complex of the target cell. In essence, a botulinum toxin light chain comprising an enzymatic domain is intracellularly delivered to any desired cell by selecting the appropriate targeting domain.

The clostridial derivative, such as a botulinum toxin, for use according to the present methods can be stored in lyophilized, vacuum dried form in containers under vacuum pressure or as stable liquids. Prior to lyophilization the botulinum toxin can be combined with pharmaceutically acceptable excipients, stabilizers and/or carriers, such as, for example, albumin, or the like. In embodiments containing albumin, the albumin can be, for example, human serum albumin, or the like. The lyophilized material can be reconstituted with a suitable liquid such as, for example, saline, water, or the like to create a solution or composition containing the botulinum toxin to be administered to the patient.

In some embodiments, the clostridial derivative is provided in a controlled release system comprising a polymeric matrix encapsulating the clostridial derivative, wherein a fractional amount of the clostridial derivative is released from the polymeric matrix over a prolonged period of time in a controlled manner. Controlled release neurotoxin systems have been disclosed for example in U.S. Patent Nos. 6,585,993; 6,585,993; 6,306,423 and 6,312,708, each of which is hereby incorporated by reference in its entirety.

In alternative embodiments, the clostridial derivative is provided in an ointment, gel, cream, or emulsion suitable for topical administration.

The therapeutically effective amount of the clostiridial derivative, for example a botulinum toxin, administered according to the present method can vary according to the potency of the toxin and particular characteristics of the pain being treated, including its severity and other various patient variables including size, weight, age, and responsiveness to therapy. The potency of the toxin is expressed as a multiple of the LD₅₀ value for the mouse, one unit (U) of toxin being defined as being the equivalent amount of toxin that kills 50% of a group of 18 to 20 female Swiss-Webster mice, weighing about 20 grams each.

The therapeutically effective amount of the botulinum toxin can vary according to the potency of a particular botulinum toxin, as commercially available Botulinum toxin formulations do not have equivalent potency units. It has been reported that one Unit of BOTOX^{®} (onabotulinumA), a botulinum toxin type A available from Allergan, Inc., has a potency Unit that is approximately equal to 3 to 5 Units of DYSPORT^{®} (abobotulinumtoxinA), also a botulinum toxin type A available from Ipsen Pharmaceuticals. MYOBLOC^{®}, a botulinum toxin type B available from Elan, has been reported to have a much lower potency Unit relative to BOTOX^{®}. In some embodiments, the botulinum neurotoxin can be a pure toxin, devoid of complexing proteins, such as XEOMIN^{®} (incobotulinumtoxinA). One unit of incobotulinumtoxinA has been reported to have potency approximately equivalent to one unit of onabotulinumtoxinA. Thus, the quantity of toxin administered and the frequency of its administration will be at the discretion of the physician responsible for the treatment and will be commensurate with questions of safety and the effects produced by a particular toxin formulation.

The dosages used in human therapeutic applications are roughly proportional to the mass of the tissue being injected. Typically, the dose of a clostridial derivative administered to the patient may be up from about 0.01 units to about 1,000 units; for example, up to about 500 units, and preferably in the range from about 80 units to about 460 units per patient per treatment, although smaller or larger doses may be administered in appropriate circumstances.

In some embodiments, the present method comprises administering a composition comprising about 10-500 units of a botulinum toxin type A, such as BOTOX^{®} to the target site. In some embodiments, the present method comprises administering a composition comprising about 25-300 units of BOTOX^{®} to the target site. In one specific embodiment, the present method comprises administering a composition comprising about 100-200 units of BOTOX^{®} to the target site. In some embodiments, the composition is administered to the target site of the bladder or its vicinity, e.g. the detrusor. In certain embodiments, the dosage can range from about 10 units to about 200 units per treatment. In some embodiments, the dosage per treatment is 20 units, 30 units, 40 units, 50 units, 60 units, 70 units, 80 units, 90 units, 100 units, 110 units, 120 units, 130 units, 140 units, 150 units, 160 units, 170 units, 180 units, 190 units or 200 units of a botulinum toxin type A, such as onabotulinumtoxinA. In alternative embodiments, the present method comprises administering a composition comprising about 75-1500 units of abobotulinumA to the target site. In one specific embodiment, the present method comprises administering a composition comprising about 300-1000 units of abobotulinumA to the target site. In some embodiments, the composition is administered to the target site of the bladder or its vicinity, *e.g.* the detrusor. In certain embodiments, the dosage can range from about 30 Units to about 1000U per treatment. In yet alternative embodiments, the present method comprises administering a composition comprising about 25-300 units of incobotulinumtoxinA to the target site. In one specific embodiment, the present method comprises administering a composition comprising about 100-200 units of incobotulinumtoxinA to the target site. In some embodiments, the composition is administered to the target site of the bladder or its vicinity, e.g. the detrusor. In certain embodiments, the dosage can range from about 10 units to about 200 units of incobotulinumtoxinA per treatment. In some embodiments, if the neurotoxin is botulinum toxin type B, the dosage is approximately 50 times greater than the functionally equivalent dosage of botulinum toxin type A.

The method comprises administering the composition comprising the clostridial derivative to a target site below (or inferior to) the bladder midline. In one embodiment, the target site is a single site of administration below the bladder midline. In other embodiments, the target site comprises a plurality of administration sites. In some embodiments, the pharmaceutical composition is administered to a plurality of target sites, or at multiple target sites, ranging from 2 sites or 3 sites up to about 50 sites, or from 2 sites or 3 sites to 40 sites, or from 2 sites or 3 sites to 30 sites, or from 2 sites or 3 sites to 20 sites. In other embodiments, the method comprises administering to a target site, where the target site is from 1-50, 1-40, 1-30 or 1-20 sites. In other embodiments, the pharmaceutical composition is administered to 2, 3, 4, 6, 8, 10, 12, 14, 16, 18, 20, 22, 24, 26, 28, 30, 32, 34, 36, 38, 40, 42, 44, 46, 48, or 50 sites. In one embodiment, the pharmaceutical composition is administered to 20 sites. In another embodiment, the pharmaceutical composition is administered to 10 sites. In some embodiments, the target sites are all located below the bladder midline. In some embodiments, the target sites are located below the bladder midline and/or above the bladder midline.

In some embodiments, the pharmaceutical composition can be administered in multiple volumes, ranging from 0.05 mL up to 2 mL. In some embodiments, the volume of the pharmaceutical composition administered is 0.5ml/injection site. In alternative embodiments, the volume of the pharmaceutical composition administered is 0.1 mL, 0.2 mL, 0.25 mL, 0.3 mL, 0.4 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL or 2.0 mL per injection site.

In some embodiments, the method comprises administering the pharmaceutical composition to 20 sites at 0.5 mL volume per site. In some embodiments, the 20 sites are located at or below the bladder line. In some embodiments, the 20 sites are located in the lower portion of the posterior wall of the bladder which encompasses the region below the bladder line. In one embodiment, the 20 sites exclude the trigone, the dome, or combinations of both. In one embodiment, the 20 sites exclude the trigone. In some embodiments, the method comprises administering the pharmaceutical composition to 10 sites at 0.5 mL per injection site. In some embodiments, the 10 sites are located in the lower portion of the posterior wall of the bladder which encompasses the region below the bladder midline. In one embodiment, the 10 sites include eight sites in the lower portion of the posterior wall and 2 sites in the trigone. In some embodiments, the 10 sites exclude the trigone. In some embodiments, 0.1 mL is administered per injection site. In another embodiment, 0.25 mL is administered per injection site.

In some embodiments, the method consists of administering a pharmaceutical composition comprising 100 units of a botulinum toxin type A to 20 sites at 0.5 mL volume per site. In some embodiments, the method consists of administering a pharmaceutical composition comprising 100 units of onabotulinumtoxinA to 20 sites at 0.5 mL volume per site. In some embodiments, the 20 sites are located below the bladder midline. In some embodiments, the 20 sites are located in the lower portion of the posterior wall of the bladder. In one embodiment, the 20 sites exclude the trigone, the dome, or combinations of both. In another embodiment, the 20 sites are located in the posterior wall of the bladder. In one embodiment, the 20 sites exclude the trigone. In some embodiments, the method consists of administering the pharmaceutical composition comprising 100 units of a botulinum toxin type A to 10 sites at 0.5 mL per injection site. In some embodiments, the method consists of administering a pharmaceutical composition comprising 100 units of onabotulinumtoxinA to 10 sites at 0.5 mL volume per site. In some embodiments, the 10 sites are located in the lower portion of the posterior wall of the bladder. In one embodiment, the 10 sites include eight sites in the lower portion of the posterior wall and 2 sites in the trigone. In some embodiments, the 10 sites exclude the trigone. In some embodiments, 0.1 mL is administered per injection site. In another embodiment, 0.25 mL is administered per injection site.

In one embodiment, the method comprises administering a composition comprising a clostridial derivative to a plurality of target sites, where the plurality of target sites consists of 10 sites. In one embodiment, administering to the 10 target sites comprises administering the composition to 8 target sites in the posterior wall below the midline of the bladder and to 2 sites in the trigone. In one embodiment, the administering is via injection. In one embodiment, each target site in the plurality is administered 0.5 mL of the composition comprising the clostridial derivative, where the total dose of clostridial derivative is 100 units.

In another embodiment, the method comprises administering a composition comprising a clostridial derivative to a plurality of target sites, where the plurality of target sites consists of between about 5-30 sites or 10-20 sites. In one embodiment, administering to the plurality of target sites comprises administering a first part of the plurality of target sites to the posterior wall below the midline of the bladder and a second part of the plurality of target sites in the trigone. In one embodiment, the first part of the plurality of target sites is larger (or greater) in number than the second part of the plurality of target sites. In one embodiment, the administering is via injection. In one embodiment, each target site in the plurality is administered 0.2 mL, 0.3 mL, 0.4 mL, 0.5 mL, 0.6 mL, 0.7 mL or 0.8 mL of the composition comprising the clostridial derivative. In another embodiment, each target site in the plurality of target sites is administered between about 0.1-1.0 mL of a composition comprising a clostidial derivative, or between about 0.2-1.0 mL, or between about 0.2-0.9 mL, 0.2-0.8 mL, 0.3-0.8 mL, 0.3-0.7 mL, 0.4-0.8 mL, or 0.4-0.7 mL. In another embodiment, the total dose of clostridial derivative is between about 50-200 units, 50-150 units, 75-150 units, 75-125 units, 80-120 units or 90-110 units.

The treatment effects of the clostridial derivative can persist for between about 1 month and 5 years. Administration can be repeated as necessary. As a general guideline, botulinum toxin type A administered into or near muscle tissue has been observed to produce flaccid paralysis at target site muscles for up to about 3 to 6 months. However, increased efficacy of the treatment using botulinum toxin type A is expected to happen when the toxin is administered according to the disclosed method at about 3 month intervals.

In another aspect, the present disclosure provides a method for reducing the need for clean intermittent catherization (CIC) associated with overactive bladder or neurogenic detrusor overactivity treatment using a clostridial derivative in a patient. The method comprises locally administering a composition comprising a therapeutically effective amount of the clostridial derivative to a target site below (or inferior to) the bladder midline. In some embodiments, the target site comprises the bladder base, the bladder neck only, the trigone only, the posterior and lateral bladder wall, or combinations thereof. In some embodiments, the target site comprises the bladder neck only. In some embodiments, the target site comprises the posterior and lateral bladder wall. In some embodiments, the target site comprises the trigone. In alternative embodiments, the target site excludes the trigone. In some embodiments, the bladder dysfunction is an overactive bladder. In alternative embodiments, the detrusor overactivity is associated with a neurologic condition. In some embodiments, the clostridial derivative is a botulinum toxin. In some embodiments, the clostridial derivative is a botulinum toxin type A.

Example 1 describes treatment of a cohort of patients in accord with the method described herein. The patients suffered from a bladder dysfunction and were treated with botulinum toxin type A administered at one or more target sites below the bladder midline. After treatment, only 1.5% of the patients required CIC, which is a significant decrease relative to prior studies where 6.5% of patients required CIC. Thus, in one embodiment, the methods described herein provide for less than about 6%, less than about 5%, less than about 4%, less than about 3%, less than about 2% or less than about 1.5% of patients treated in accord with the method require CIC. Examples 2-5 set forth below detail additional beneficial patient outcomes provided by the treatment method herein.

A method within the scope of the present disclosure can provide improved patient function. "Improved patient function" can be defined as an improvement measured by factors such as a reduced pain, increased ambulation, healthier attitude, more varied lifestyle and/or healing permitted by normal muscle tone and function. Improved patient function may be measured with an improved quality of life (QOL) or Health-Related Quality of Life (HRQL). Scores obtained can be compared to published values available for various general and patient populations.

The following non-limiting examples provide those of ordinary skill in the art with specific preferred methods to treat urological dysfunctions such as overactive bladder or detrusor overactivity within the scope of the present disclosure, and it is not intended to limit the scope of the invention. In the following examples various modes of non-systemic administration of a botulinum neurotoxin can be carried out. For example, by intramuscular injection, non-intramuscular injection or by implantation of a controlled release implant.

### EXAMPLES

The following non-limiting examples provide those of ordinary skill in the art with specific preferred methods to treat conditions within the scope of embodiments of the present invention and are not intended to limit the scope of the invention.

### Example 1

Six healthcare professionals specialized in treatment of idiopathic overactive bladder treated 325 patients by injecting a botulinum toxin type A, Botox^{®}, into the detrusor muscle. The six practitioners limited the injection sites to below the bladder midline, specifically targeting the bladder base, the posterior and lateral bladder wall, or both. Of the 325 patients, only 5 patients have gone on to develop a post void residual urine volume that required initiation of self-catheterization. This represents a 1.5% urinary retention rate, which represents a 77% drop in urinary retention as compared to the retention rate of 6.5% previously obtained in a phase III clinical trial.

### Example 2

A 46 year old female patient is referred to the urology clinic for treatment of her non-neurogenic overactive bladder symptoms. This patient fails and/or is intolerant of numerous oral anticholinergic medications for control of her incontinence. Unfortunately, she is experiencing severe dry mouth, constipation and limited efficacy of the anticholinergic medications. The referred urologist is recommending that botulinum toxin type A (Botox^{®}) be used to treat her overactive bladder symptoms. A solution containing botulinum toxin type A is reconstituted according to the manufacturer's instructions (Botox^{®}, Allergan, Inc.) and 100 units are injected into 20 sites (10 sites in the bladder base including 2 in the trigone and 10 sites in the posterior-lateral wall the bladder midline). The patient does not experience urinary retention and her overactive bladder symptoms improve within a week and last for 6 months.

### Example 3

A 55 year old female patient is referred to the urology clinic for treatment of her neurogenic overactive bladder symptoms. The referred urologist is recommending that botulinum toxin type A (Botox^{®}) be used to treat her symptoms. A solution containing botulinum toxin type A ( is reconstituted according to the manufacturer's instructions (Botox^{®}, Allergan, Inc.) and 200 units are injected into 30 sites per approved injection paradigm. The patient comes back two weeks later and has a post-void residual volume of 350 mL and has to perform CIC for 3 months. Ten months following her treatment, she returns to her urologist's office for re-treatment but fears having to go into retention again. For her second treatment, the urologist reconstitutes the botulinum toxin type A according to the manufacturer's instructions (Botox^{®}, Allergan, Inc.) and injects 200 units into 30 sites per the proposed paradigm (15 sites in the bladder base (including 2 in the trigone) and 15 sites at and below the bladder midline in the postero-lateral wall). This time the patient does not experience urinary retention and reports a significant improvement in her quality of life.

### Example 4

A 40 year old female patient is referred to the urology clinic for treatment of her neurogenic overactive bladder symptoms. The referred urologist is recommending that botulinum toxin type A (Botox^{®}) be used to treat her symptoms. A solution containing botulinum toxin type A is reconstituted according to the manufacturer's instructions (Botox^{®}, Allergan, Inc.) and 200 units are injected into 30 sites per the prior art injection paradigm. The patient comes back two weeks later and has a post-void residual volume of 450 mL and has to perform CIC for 3 months. Ten months following her treatment, she returns to her urologist's office for re-treatment but fears having to go into retention again. For her second treatment, the urologist reconstitutes the botulinum toxin type A according to the manufacturer's instructions (Botox^{®}, Allergan, Inc.) and injects 100 units into 20 injection sites, each site in a target site below the bladder midline. This time the patient does not experience urinary retention and reports a significant improvement in her quality of life.

### Example 5

A 35 year old female patient is referred to the urology clinic for treatment of her neurogenic overactive bladder symptoms. The referred urologist is recommending that botulinum toxin type A (Botox^{®}) be used to treat her symptoms. A solution containing botulinum toxin type A is reconstituted according to the manufacturer's instructions (Botox^{®}, Allergan, Inc.) and 200 units are injected into 30 sites per the prior art injection paradigm. The patient comes back two weeks later and has a post-void residual volume of 450 mL and has to perform CIC for 3 months. Ten months following her treatment, she returns to her urologist's office for re-treatment but fears having to go into retention again. For her second treatment, the urologist reconstitutes the botulinum toxin type A according to the manufacturer's instructions (Botox^{®}, Allergan, Inc.) and injects 100 units into 10 injection sites, where each injection site is in a target site below the bladder midline, wherein 8 sites are in the lower portion of the posterior wall of the bladder and 2 sites are in the trigone. This time the patient does not experience urinary retention and reports a significant improvement in her quality of life.

Many alterations and modifications may be made by those having ordinary skill in the art, without departing from the spirit and scope of the disclosure. Therefore, it must be understood that the described embodiments have been set forth only for the purposes of examples, and that the embodiments should not be taken as limiting the scope of the following claims. The following claims are, therefore, to be read to include not only the combination of elements which are literally set forth, but all equivalent elements for performing substantially the same function in substantially the same way to obtain substantially the same result. The claims are thus to be understood to include those that have been described above, those that are conceptually equivalent, and those that incorporate the ideas of the disclosure.

The present invention may be summarized by reference to the following embodiments (embs.):
Emb. 1. Use of a composition comprising a therapeutically effective amount of a clostridial derivative for the treatment of a bladder dysfunction or detrusor overactivity in a patient, the composition administered to a target site inferior to a bladder midline.
Emb. 2. Use of a composition comprising a therapeutically effective amount of a clostridial derivative for reducing or preventing the risk for urinary retention associated with overactive bladder or neurogenic detrusor overactivity treatment using a clostridial derivative in a patient, the composition administered to a target site inferior to a bladder midline.
Emb. 3. Use of a composition comprising a therapeutically effective amount of a clostridial derivative for reducing the need for clean intermittent catherization (CIC) associated with overactive bladder or neurogenic detrusor overactivity treatment using a clostridial derivative in a patient, the composition administered to a target site inferior to a bladder midline.
Emb. 4. The use according to Emb. 1, wherein the bladder dysfunction is overactive bladder.
Emb. 5. The use according to any one of Embs. 1-3, wherein the detrusor overactivity is associated with a neurologic condition.
Emb. 6. The use according to any one of Embs. 1-5, wherein the target site comprises the bladder base, the posterior and lateral bladder wall below the midlineor both.
Emb. 7. The use according to any one of Embs. 1-6, wherein the therapeutically effective amount is administered at multiple target sites.
Emb. 8. The use according to any one of Embs. 1-7, wherein the clostridial derivative is a botulinum toxin.
Emb. 9. The use according to Emb. 8, wherein the botulinum toxin is selected from the group consisting of botulinum toxin types A, B, C, D, E, F and G.
Emb. 10. The use according to Emb. 8 or Emb. 9, wherein the botulinum neurotoxin is type A.
Emb. 11. The use according to any one of Embs. 1-8, wherein the therapeutically effective amount is between about 10 units to about 500 units.
Emb. 12. The use according to any preceding Emb. wherein the bladder midline corresponds to an imaginary approximately 2-3 cm above an imaginary line intersecting the trigone above the ureteral orifices.
Emb. 13. The use according to any preceding Emb., wherein the administering is by injection.
Emb. 14. The use according to Emb. 13, wherein the administering is by injection to the detrusor muscle.

## Claims

1. A composition comprising a therapeutically effective amount of a clostridial derivative for use in the treatment of a bladder dysfunction or detrusor overactivity in a patient, wherein the composition is administered to a target site inferior to a bladder midline.

2. A composition comprising a therapeutically effective amount of a clostridial derivative for use in reducing or preventing the risk for urinary retention associated with overactive bladder or neurogenic detrusor overactivity treatment in a patient, wherein the composition is administered to a target site inferior to a bladder midline.

3. A composition comprising a therapeutically effective amount of a clostridial derivative for use in reducing the need for clean intermittent catherization (CIC) associated with overactive bladder or neurogenic detrusor overactivity treatment in a patient, wherein the composition is administered to a target site inferior to a bladder midline.

4. The composition for use according to claim 1, wherein the bladder dysfunction is overactive bladder.

5. The composition for use according to any one of claims 1-3, wherein the detrusor overactivity is associated with a neurologic condition.

6. The composition for use according to any one of claims 1-5, wherein the target site comprises the bladder base, the posterior and lateral bladder wall below the midlineor both.

7. The composition for use according to any one of claims 1-6, wherein the therapeutically effective amount is administered at multiple target sites.

8. The composition for use according to any one of claims 1-7, wherein the clostridial derivative is a botulinum toxin.

9. The composition for use according to claim 8, wherein the botulinum toxin is selected from the group consisting of botulinum toxin types A, B, C, D, E, F and G.

10. The composition for use according to claim 8 or claim 9, wherein the botulinum neurotoxin is type A.

11. The composition for use according to any one of claims 1-8, wherein the therapeutically effective amount is between about 10 units to about 500 units.

12. The composition for use according to any preceding claim wherein the bladder midline corresponds to an imaginary approximately 2-3 cm above an imaginary line intersecting the trigone above the ureteral orifices.

13. The composition for use according to any preceding claim, wherein the administering is by injection.

14. The composition for use according to claim 13, wherein the administering is by injection to the detrusor muscle.
